Europäisches Patentamt

European Patent Office

Office européen des brevets

(19)

(11) Veröffentlichungsnummer: **0 299 910**
A2

## (12) EUROPÄISCHE PATENTANMELDUNG

(21) Anmeldenummer: 88730158.8

(22) Anmeldetag: 13.07.88

(51) Int. Cl.⁴: **C 07 H 11/00**
A 61 K 49/00, A 61 K 49/04

(30) Priorität: 17.07.87 DE 3724188

(43) Veröffentlichungstag der Anmeldung:
18.01.89 Patentblatt 89/03

(84) Benannte Vertragsstaaten:
AT BE CH DE ES FR GB GR IT LI LU NL SE

(71) Anmelder: SCHERING AKTIENGESELLSCHAFT Berlin
und Bergkamen
Müllerstrasse 170/178 Postfach 65 03 11
D-1000 Berlin 65 (DE)

(72) Erfinder: Gries, Heinz, Dr.
Helmstedter Strasse 19
D-1000 Berlin 31 (DE)

Schmitt-Willich, Heribert, Dr.
Triftstrase 39
D-1000 Berlin 39 (DE)

Speck, Ulrich, Prof. Dr.
Benediktiner Strasse 50
D-1000 Berlin 28 (DE)

Mützel, Wolfgang, Dr.
Weddigenweg 74
D-1000 Berlin 45 (DE)

(54) Metallhaltige Oligosaccharid-Polysulfate, Verfahren zu deren Herstellung und diese enthaltende pharmazeutische Mittel.

(57) Metallhaltige Oligosaccharid-Polysulfat-Verbindungen, en-haltend mindestens ein Metallion eines Elements der Ordnungszahlen 21 bis 29, 42, 44 oder 56 bis 83 sind hervorragend geeignet zur Erkennung gastrointestinaler Erkrankungen.

EP 0 299 910 A2

**Beschreibung**

### Metallhaltige Oligosaccharid-Polysulfate, Verfahren zu deren Herstellung und diese enthaltende pharmazeutische Mittel

Die Erfindung betrifft den in den Patentansprüchen gekennzeichneten Gegenstand, das heißt neue metallhaltige Oligosaccharid-Polysulfat-Verbindungen, diese Verbindungen enthaltende Mittel, ihre Verwendung als Diagnostika sowie Verfahren zur Herstellung dieser Verbindungen und Mittel.

Schätzungsweise mindestens 10 % der Bevölkerung in den westlichen Industrieländern leidet unter gastrointestinalen Erkrankungen. Die meisten dieser Patienten sowie eine große Anzahl von Patienten bei denen der Verdacht auf eine solche Erkrankung vorliegt müssen sich diagnostischen Untersuchungen unterziehen. Zur Zeit sind vor allem zwei dafür geeignete Methoden gebräuchlich: Die Endoskopie und die Röntgenstrahl-Diagnostik mit Hilfe von Barium-Kontrastmitteln.

Diese Untersuchungen sind mit den verschiedensten Nachteilen behaftet: sie sind trauma-verursachend, mit Unannehmlichkeiten und Belastungen, gelegentlich sogar mit Risiken für den Patienten verbunden, können daher psychologischen Streß hervorrufen, sind zur Entdeckung eines kleinen Ulcus oft nicht empfindlich genug, müssen meistens wiederholt durchgeführt werden, sind relativ aufwendig durchzuführen, erfordern die aktive Mitarbeit des Patienten (z.B. Einnehmen einer bestimmten Körperhaltung) und sind oft nicht anwendbar bei gebrechlichen und bei Risiko-Patienten.

Der Erfindung liegt somit die Aufgabe zugrunde, neue diagnostische Methoden, die diese Nachteile nicht besitzen, zur Verfügung zu stellen.

Diese Aufgabe wird durch die Erfindung, die neue Verbindungen und Mittel für die Erkennung und Lokalisierung gastrointestinaler Erkrankungen sowie Verfahren zu deren Herstellung betrifft, gelöst.

Es wurde gefunden, daß sich metallhaltige Oligosaccharid-Polysulfat-Verbindungen, die mindestens ein Metallion eines Elements der Ordnungszahlen 21-29, 42, 44 oder 56 bis 83 enthalten, überraschenderweise hervorragend zur Herstellung von NMR- und Röntgen-Diagnostika für die Erkennung von gastrointestinalen Erkrankungen eignen.

Die erfindungsgemäßen Mittel sind auf Grund ihrer guten Akzeptanz durch den Patienten, ihrer hohen Empfindlichkeit und ihrer ulcus-spezifischen Bindung im Gastrointestinaltrakt vor allem auch zur Früherkennung sowie für Nachuntersuchungen bei der Therapie dieser Erkrankungen verwendbar.

Das Element der oben genannten Ordnungszahl, darf für den angestrebten Verwendungszweck des erfindungsgemäßen diagnostischen Mittels nicht radioaktiv sein.

Ist das erfindungsgemäße Mittel zur Anwendung in der NMR-Diagnostik bestimmt, so muß das Metallion der Oligosaccharid-Polysulfat-Verbindung paramagnetisch sein. Dies sind insbesondere die zwei- und dreiwertigen Ionen der Elemente der Ordnungszahlen 21 bis 29, 42, 44 und 57 bis 70. Geeignete Ionen sind beispielsweise das Chrom(III)-, Mangan(II)-, Eisen(II)-, Cobalt(II)-, Nickel(II)-, Kupfer(II)-, Praseodym(III)-, Neodym(III)-, Samarium(III)- und Ytterbium(III)-ion. Wegen ihres sehr starken magnetischen Moments sind besonders bevorzugt das Gadolinium(III)-, Terbium(III)-, Dysprosium(III)-, Holmium(III)-, Erbium(III)-und Eisen(III)-ion.

Ist das erfindungsgemäße Mittel zur Anwendung in der Röntgen-Diagnostik bestimmt, so muß sich das Zentralion von einem Element höherer Ordnungszahl ableiten, um eine ausreichende Absorption der Röntgenstrahlen zu erzielen. Es wurde gefunden, daß zu diesem Zweck diagnostische Mittel, die ein Metallion vor Elementen der Ordnungszahlen zwischen 56 bis 83 enthalten, besonders geeignet sind; dies sind beispielsweise das Lanthan(III)-ion und die oben genannten Ionen der Lanthanidenreihe.

Als Oligosaccharide werden bevorzugt Saccharose, Laktose, Maltose und Trehalose verwendet.

Die Herstellung der neuen Verbindungen erfolgt erfindungsgemäß in der Weise, daß man das Metalloxid oder ein Metallsalz (beispielsweise das Nitrat, Acetat, Carbonat, Chlorid oder Sulfat) des Elements der Ordnungszahlen 21 bis 27, 42, 44 oder 56 bis 83 mit dem gewünschten Oligosaccharid-Polysulfat, gegebenenfalls in Form eines Salzes, umsetzt.

Als bevorzugte Oligosaccharid-Polysulfat-Salze seien solche, die Kationen anorganischer und/oder organischer Basen oder Aminosäuren, wie zum Beispiel Ammonium, Lithium, Natrium, Kalium, Barium, Calcium und Magnesium bzw. von primären, sekundären oder tertiären Aminen, wie zum Beispiel Ethanolamin, Diethanolamin, Morpholin, Glucamin, N.N-Dimethylglucamin und insbesondere N-Methylglucamin sowie Kationen von Aminosäuren wie beispielsweise die des Lysins, des Arginins und des Ornithins enthalten, genannt.

Es können auch mehr als ein Metalloxid oder Metallsalz unterschiedlicher Elemente in die Reaktion eingesetzt werden, so daß die gebildete erfindungsgemäße Verbindung unterschiedliche Metallionen im Molekül enthält.

Als für den angegebenen Zweck geeignet haben sich auch diagnostische Mittel erwiesen, die als Oligosaccharid-Polysulfat-Verbindung Sucralfat, ein Sucralfat-Derivat oder eine SucralfatVorstufe enthalten. Das für die Bildgebung benötigte Metallion liegt dabei vorzugsweise in Form von Verbindungen, wie sie zum Beispiel in den Patentschriften EP 71564; EP 130934; US-4,719,098; US-4,647,447; US-4,637,929 und US-4,731,239 beschrieben sind, vor.

Die Umsetzung wird in wäßriger Lösung durch Mischen der beiden Komponenten Metalloxid- bzw. Metallsalz-Lösung und Oligosaccharid-Polysulfat(-salz) unter Rühren bei Raumtemperatur in einem pH-Bereich von 3,0 bis 5,5 durchgeführt. Die Einstellung des erforderlichen pH-Bereichs erfolgt durch Zugabe solcher Säuren bzw. Basen, die die Reaktion nicht beeinträchtigen. Bevorzugt sind Salzsäure bzw. Natronlauge.

Die Isolierung der erfindungsgemäßen Verbindungen erfolgt durch Filtrieren oder Zentrifugieren, Waschen und Trocknen des bei der Umsetzung entstehenden Präzipitats. Die Nichteinhaltung des angegebenen pH-Bereichs bewirkt eine Ausbeuteminderung oder das Nichtauftreten des gewünschten Präzipitats.

Bei zu hoher Konzentration der Reaktionspartner kann ebenfalls die Bildung eines Niederschlags ausbleiben. In diesem Fall kann durch Verdünnen mit Wasser der gewünschte Effekt hervorgerufen werden.

In vielen Fällen tritt, bei Beachtung der oben genannten Bedingungen, sofort beim Mischen der Reaktionspartner ein Niederschlag auf. Zur Vervollständigung der Reaktion, zur Ausbeuteverbesserung sowie zur Bildung besser filtrierbarer Kristalle ist es vorteilhaft die Reaktionszeit über mehrere Stunden, zum Beispiel 4 bis 16 Stunden, zu erstrecken.

Die Herstellung der Edukte, das heißt der Oligosaccharid-Polysulfate sowie deren Salze, erfolgt nach literaturbekannten Verfahren Umsetzung eines sulfatgruppen-einführenden Reagenzes mit einem Oligosaccharid wie Saccharose, Laktose, Maltose oder Trehalose.

Geeignete Reagenzien sind zum Beispiel Schwefelsäure, Chlorsulfonsäure und Schwefeltrioxid-Pyridin, die in Lösungsmitteln wie Chloroform, Dimethylformamid, Formamid, flüssigem Schwefeltrioxid oder Pyridin eingesetzt werden. Bei Verwendung des bevorzugten Reagenzes Schwefeltrioxid-Pyridin wird das gewünschte Disaccharid bei ca. 50-70°C 3 bis 7 Stunden mit diesem Komplex umgesetzt. Will man die so erhaltene Lösung des Oligosaccharid-Polysulfats direkt, das heißt ohne Isolierung dieses Edukts, in die nachfolgende Reaktion zur Bildung der erfindungsgemäßen metallhaltigen Verbindungen einsetzen, so muß zunächst die stark saure Lösung in einen basischeren Bereich überführt werden. Man benutzt dazu zum Beispiel Natrium-, Calcium-, Barium- oder Magnesiumhydroxid.

Die so erhaltenen Oligosaccharid-Polysulfat-Edukte weisen als Natriumsalz einen Schwefelgehalt von im allgemeinen 7-21 % auf.

Die Herstellung der erfindungsgemäßen diagnostischen Mittel erfolgt in an sich bekannter Weise, indem man die erfindungsgemäßen Verbindungen gegebenenfalls unter Zugabe der in der Galenik üblichen Zusätze in wäßrigem Medium suspendiert und anschließend die Suspension gegebenenfalls sterilisiert. Geeignete Zusätze sind beispielsweise physiologisch unbedenkliche Puffer (wie z.B. Tromethamin) oder, falls erforderlich, Elektrolyte wie zum Beispiel Natriumchlorid oder, falls erforderlich, Antioxidantien wie zum Beispiel Ascorbinsäure oder andere Stabilisatoren oder auch Gasbildner wie zum Beispiel Bicarbonat.

Sind für die enterale Verabreichung oder andere Zwecke Suspensionen der erfindungsgemäßen Mittel in Wasser oder physiologischer Salzlösung erwünscht, können sie mit einem oder mehreren in der Galenik üblichen Hilfsstoffen (z.B. Methylcellulose, Lactose, Mannit) und/oder Tensiden (z.B. Lecithine, Tweens[R], Myrj[R]) und/oder Aromastoffen zur Geschmackskorrektur (z.B. ätherischen Ölen) und/oder Substanzen zur Beeinflussung der Haftfähigkeit der Wirkstoffe an den Schleimhäuten (z.B. Aminosäuren und deren Derivate) gemischt werden.

Für die bevorzugte orale Applikation kommen insbesondere Tabletten, Dragées, Kapseln, Pillen, Suspensionen oder Lösungen infrage, die in üblicher Weise mit den in der Galenik gebräuchlichen Zusätzen und Trägersubstanzen hergestellt werden können.

Die erfindungsgemäßen NMR-diagnostischen Mittel enthalten 0,05 bis 500 mMol, vorzugsweise 0,5 bis 20 mMol, der erfindungsgemäßen Verbindung pro Liter und werden in der Regel in Mengen von 0,001 bis 5mMol/kg dosiert. Für die Röntgendiagnostik werden eher höhere Konzentrationen und Dosierungen eingesetzt. Sie sind zur enteralen Applikation bestimmt.

Die erfindungsgemäßen Verbindungen kommen zur Anwendung

1) für die NMR-Diagnostik, wenn sie Metallionen der Elemente mit den Ordnungszahlen 21 bis 29, 42, 44 und 57 bis 70,

2) für die Rontgendiagnostik, wenn sie Metallionen der Elemente der Ordnungszahlen 56 bis 83 enthalten.

Die erfindungsgemäßen Mittel erfüllen die vielfältigen Voraussetzungen für die Eignung als Kontrastmittel für die Kernspintomographie. So sind sie hervorragend dazu geeignet, nach enteraler Applikation durch selektive Erhöhung der Signalintensität an Orten pathologischer Veränderungen der Schleimhäute das mit Hilfe des Kernspintomographen erhaltene Bild in seiner Aussagekraft zu verbessern. Ferner zeigen sie die hohe Wirksamkeit, die notwendig ist, um den Körper mit möglichst geringen Mengen an Fremdstoffen zu belasten, und die gute Verträglichkeit, die notwendig ist, um den nichtinvasiven Charakter der Untersuchungen aufrechtzuerhalten.

Im allgemeinen werden die erfindungsgemäßen Mittel für die Anwendung als NMR-Diagnostika in Mengen von 0,001 bis 5 mMol Metall/kg Körpergewicht, vorzugsweise 0,005 bis 0,5 mMol Metall/kg, dosiert. Details der Anwendung werden zum Beispiel in H.J. Weinmann et al, Am J. of Roentgenology 142, 619 (1984) diskutiert.

Für die Anwendung als Röntgenkontrastmittel werden die erfindungsgemäßen Mittel in Mengen von 0,01 bis 10 mMol Metall/kg, vorzugsweise von 0,1 bis 2 mMol Metall/kg dosiert.

Details der Anwendung von Röntgenkontrastmittel werden zum Beispiel in Barke "Röntgenkontrastmittel" W. Thieme, Leipzig (1970) und P. Thurn, E. Bücheler "Einführung in die Röntgendiagnostik", W. Thieme, Stuttgart, New York (1977) diskutiert.

Die nachfolgenden Beispiele dienen zur Erläuterung des Erfindungsgegenstandes.

Beispiel 1

11,6 g (= 10 mMol) Natriumsalz des Saccharose-octasulfats (hergestellt nach US 3432489), werden in einer Lösung von 39,6 g (= 80 mmol) Gadoliniumcarbonat, $Gd_2(CO_3)_3$, in 500 ml einer 1n-Salzsäure gelöst. Dann bringt man durch langsames Zutropfen von 1n-Natronlauge den Ansatz auf $P_H$ 4,5 bis 5,0 und hält ihn durch weiteres langsames Zutropfen von 1n-Natronlauge solange in diesem $P_H$-Bereich, bis keine weitere Trubung mehr auftritt. Nach Stehen über Nacht saugt man den Niederschlag über ein feinporiges Filter ab, wäscht ihn mit Wasser chloridfrei und trocknet ihn im Vakuum bei 60°C.

Man erhält 20 g der Saccharosepolysulfat-Gadoliniumverbindung als weisses Pulver mit einem Gadoliniumgehalt von 45,39 Gew. %.

In analoger Weise erhält man aus dem Kaliumsalz des Maltose-polysulfats und des Natriumsalzes des Lactose-polysulfats die entsprechenden Gadoliniumverbindungen.

Verwendet man anstelle von Gadoliniumcarbonat Ytterbiumcarbonat, so erhält man die entsprechenden Disaccharid-Polysulfat-Ytterbiumverbindungen.

Beispiel 2

10 ml einer wäßrigen Suspension mit der in Beipsiel 1 beschriebenen Verbindung für die enterale Applikation enthalten:
0,52 g Saccharose-polysulfat-Gadolinium
0,05 g Natriumdihydrogenphosphat
0,01 g Xanthangummi
0,65 g Glycerin
Wasser ad 10 ml

Beispiel 3

Zusammensetzung einer Tablette mit Saccharosepolysulfat-Gadolinium zur oralen Applikation

```
250 mg  Saccharosepolysulfat-Gadolinium
 82 mg  Laktose
 50 mg  Maisstärke
 10 mg  Magnesiumstearat
  0,5   Natriumcarboxymethylcellulose
  7,5   Calciumcarboxymethylcellulose
400 mg  Gesamtgewicht
```

Die drei erstgenannten Bestandteile werden vermischt und mit einer Paste aus Natriumcarboxymethylcellulose (hergestellt durch Vorbehandlung mit Ethanol und Wasser) versetzt. Nach Verreiben und Granulieren wird getrocknet, mit Calciumcarboxymethylcellulose und Magnesiumstearat vesetzt und anschließend maschinell zu einer Tablette formuliert.

Beispiel 4

Zusammensetzung eines Granulats mit Saccharosepolysulfat-Ytterbium zur oralen Applikation

```
500 mg Saccharosepolysulfat-Ytterbium

244 mg Maisstärke

  4 mg Methylcellulose

  2 mg 1-Menthol

750 mg Gesamtgewicht
```

Zu einer pulverförmigen Mischung aus Saccharosepolysulfat und Maisstärke wird ein Binder, hergestellt durch Dispersion von Methylcellulose in einer Lösung von 1-Methanol in Ethanol und Wasser, gegeben. Nach intensivem Verreiben wird das Gemisch maschinell zu einem Granulat formuliert und getrocknet.

### Beispiel 5

4,6 g (4 mmol) Natriumsalz des Saccharose-octasulfats (hergestellt nach US 3432489) werden in einer Lösung von 17,3 g (64 mmol) Eisen(III)-chlorid $FeCl_3$ x $6H_2O$ gelöst. Dann bringt man durch langsames Zutropfen von 330 ml einer 0,5 n-Natronlauge die entstandene Suspension auf pH = 3.0. Nach Stehen über Nacht saugt man den Niederschlag über ein feinporigen Filter ab, wäscht ihn mit Wasser chloridfrei und trocknet ihn im Vakuum bei 60° C.
Man erhält 8,0 g der Saccharosepolysulfat-Eisenverbindung als braunes Pulver mit einem Eisengehalt von 38,2 Gew%.
In analoger Weise erhält man aus dem Kaliumsalz des Maltose-polysulfats und des Natriumsalzes des Lactose-polysulfats die entsprechenden Eisenverbindungen.

### BEISPIEL 6

8,0 mg der nach Beispiel 5 erhaltenen Saccharosepolysulfat-Eisenverbindung mit dem Eisengehalt von 38,2 Gew.% werden mit 5 ml der käuflich erhältlichen Ulcogant(R)-Suspension (E. Merck, Darmstadt) unter Rühren bei Raumtemperatur gemischt. Die so erhaltene Suspension ist für die enterale Applikation verwendbar.

### BEISPIEL 7

174 mg der nach Beispiel 1 erhaltenen Saccharosepolysulfat-Gadoliniumverbindung mit dem Gadoliniumgehalt von 45,39 Gew.% werden mit 5 ml der käuflich erhältlichen Ulcogant(R)-Suspension (E. Merck, Darmstadt) unter Rühren bei Raumtemperatur gemischt. Die so erhaltene Suspension ist für die enterale Applikation verwendbar.

### Patentansprüche

1) Metallhaltige Oligosaccharid-Polysulfat-Verbindungen, enthaltend mindestens ein Metallion eines Elements der Ordnungszahlen 21 bis 29, 42, 44 oder 56 bis 83.

2) Verbindungen gemäß Anspruch 1, dadurch gekennzeichnet, daß sie als Oligosaccharid Saccharose, Laktose, Maltose oder Trehalose enthalten.

3) Verbindungen gemäß Anspruch 1, dadurch gekennzeichnet, daß sie als Metallion Gadolinium oder Ytterbiumion enthalten.

4) Diagnostische Mittel zur Erkennung gastrointestinaler Erkrankungen enthaltend mindestens eine Verbindung gemäß Anspruch 1, gegebenenfalls mit den in der Galenik üblichen Zusätzen.

5. Diagnostische Mittel gemäß Anspruch 4, dadurch gekennzeichnet, daß sie als Oligosaccharid-Polysulfat-Verbindung Sucralfat, ein Sucralfat-Derivat oder eine Sucralfat-Vorstufe enthalten.

6. Diagnostische Mittel gemäß Anspruch 4, dadurch gekennzeichnet, daß sie mindestens ein Metallion in Form einer Komplexverbindung enthalten.

7.) Verwendung mindestens einer Verbindung gemäß Anspruch 1 für die Herstellung von Mitteln für die NMR- und Röntgen-Diagnostik.

8.) Verfahren zur Herstellung von Verbindungen gemäß Anspruch 1, dadurch gekennzeichnet, daß man ein Oligosaccharid-Polysulfat, gegebenenfalls in Form eines Salzes mit einer Metallionen mindestens eines Elements der Ordnungszahlen 21 bis 29, 42, 44 oder 56 bis 83 enthaltenden Lösung in einem pH-Bereich zwischen 3,5 und 5,5 umsetzt.

9.) Verfahren zur Herstellung der diagnostischen Mittel gemäß Anspruch 4, dadurch gekennzeichnet, daß man die in Wasser oder physiologischer Salzlösung suspendierten Verbindungen, gegebenenfalls mit den in der Galenik üblichen Zusätzen, in eine für die enterale Applikation geeignete Form bringt.